(19) **Europäisches Patentamt / European Patent Office / Office européen des brevets**

(11) **EP 3 512 506 B1**

(12) **EUROPEAN PATENT SPECIFICATION**

(45) Date of publication and mention of the grant of the patent:
**26.01.2022 Bulletin 2022/04**

(21) Application number: **17851614.2**

(22) Date of filing: **15.09.2017**

(51) International Patent Classification (IPC):
*A61K 31/451* (2006.01)     *A61K 9/20* (2006.01)
*A61K 9/28* (2006.01)     *A61K 9/48* (2006.01)
*A61P 21/00* (2006.01)

(52) Cooperative Patent Classification (CPC):
**A61P 21/00; A61K 31/451**

(86) International application number:
**PCT/US2017/051803**

(87) International publication number:
**WO 2018/053280 (22.03.2018 Gazette 2018/12)**

(54) **USE OF PRIDOPIDINE FOR TREATING RETT SYNDROME**

VERWENDUNG VON PRIDOPIDIN ZUR BEHANDLUNG DES RETT-SYNDROMS

UTILISATION DE LA PRIDOPIDINE POUR LE TRAITEMENT DU SYNDROME DE RETT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.09.2016 US 201662395854 P**

(43) Date of publication of application:
**24.07.2019 Bulletin 2019/30**

(73) Proprietor: **Prilenia Neurotherapeutics Ltd.**
**Herzliya 4672561 (IL)**

(72) Inventors:
• **GEVA, Michal**
**Even-Yehuda (IL)**
• **LAUFER, Ralph**
**6523309 Tel Aviv (IL)**
• **HAYDEN, Michael**
**49131 Petach-Tikva (IL)**

(74) Representative: **Pearl Cohen Zedek Latzer Baratz UK LLP**
**The Gridiron Building**
**One Pancras Square**
**London N1C 4AG (GB)**

(56) References cited:
WO-A1-01/46145     WO-A1-01/46145
WO-A1-2013/086425     WO-A1-2013/086425
WO-A1-2015/112601     WO-A1-2015/112601
WO-A1-2016/138135     WO-A1-2016/138135
US-A1- 2016 243 098

• **M. Geva ET AL: "Pridopidine Treatment Recovers Gait Abnormalities and Rescues Impaired BDNF Expression in a Rett Syndrome Mouse Model (P3.324)", Neurology, 9 April 2018 (2018-04-09), pages 1-6, XP055687599, Retrieved from the Internet: URL:https://n.neurology.org/content/90/15_ Supplement/P3.324 [retrieved on 2020-04-20]**

EP 3 512 506 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

SEQUENCE LISTING

<110> Teva Pharmaceuticals International GmbH

<120> USE OF PRIDOPIDINE FOR TREATING RETT SYNDROME

<130> 88973-A-PCT/JPW/GJG/JTC

<150> 62/395,854
<151> 2016-09-16

<160> 14

<170> PatentIn version 3.5

<210> 1
<211> 18
<212> DNA
<213> Artificial Sequence

<220>
<223> 5' Primer Sequence

<400> 1
ggcacaatgc aggaaagg 18

<210> 2
<211> 21
<212> DNA
<213> Artificial Sequence

<220>
<223> 3' Primer Sequence

<400> 2
tcagcaggca catagatagc c 21

<210> 3
<211> 19
<212> DNA
<213> Artificial Sequence

<220>
<223> 5' Primer Sequence

<400> 3
ttgtggccaa gcaggtact 19

<210> 4
<211> 20
<212> DNA
<213> Artificial Sequence

<220>
<223> 3' Primer Sequence

<400> 4
gttgatgcct tcacagcgta    20

<210> 5
<211> 21
<212> DNA
<213> Artificial Sequence

<220>
<223> 5' Primer Sequence

<400> 5
caatgtgtcc gtcgtggatc t 21

<210> 6
<211> 22
<212> DNA
<213> Artificial Sequence

<220>
<223> 3' Primer Sequence

<400> 6
gtcctcagtg tagcccaaga tg 22

<210> 7
<211> 20
<212> DNA
<213> Artificial Sequence

<220>
<223> 5' Primer Sequence

<400> 7
agtctccagg acagcaaagc 20

<210> 8
<211> 23
<212> DNA
<213> Artificial Sequence

<220>
<223> 3' Primer Sequence

<400> 8
tgcaaccgaa gtatgaaata acc 23

<210> 9
<211> 22
<212> DNA

<213> Artificial Sequence

<220>
<223> 5' Primer Sequence

<400> 9
gctgccttga tgtttacttt ga 22

<210> 10
<211> 23
<212> DNA
<213> Artificial Sequence

<220>
<223> 3' Primer Sequence

<400> 10
aaggatggtc atcactcttc tca 23

<210> 11
<211> 19
<212> DNA
<213> Artificial Sequence

<220>
<223> 5' Primer Sequence

<400> 11
ccgagagctt tgtgtggac 19

<210> 12
<211> 21
<212> DNA
<213> Artificial Sequence

<220>
<223> 3' Primer Sequence

<400> 12
tcatgcaacc gaagtatgaa a 21

<210> 13
<211> 20
<212> DNA
<213> Artificial Sequence

<220>
<223> 5' Primer Sequence

<400> 13
gcctttggag cctcctctac 20

<210> 14
<211> 19
<212> DNA
<213> Artificial Sequence

<220>

<223> 3' Primer Sequence

<400> 14
gcggcatcca ggtaatttt 19

**Description**

**BACKGROUND**

Rett Syndrome

Rett syndrome (RTT) is a neurological disorder estimated to affect 1 in every 10,000 to 15,000 live female births in all racial and ethnic groups. (Amaral 2007).

In 95%-97% of cases, RTT is caused by a mutation in the Methyl-CpG binding Protein 2 (MeCP2) gene located on the X chromosome. (Isaias 2014). The mutation is usually random and spontaneous. In less than 1% of recorded cases, the mutation is inherited or passed from one generation to the next. The MeCP2 gene is involved in the production of the methylcystine binding protein 2 (MeCP2) protein. The MeCP2 protein binds methylcytosine and 5-hydroxymethycytosine at CpG sites in promoter regions of target genes, controlling their transcription by recruiting co-repressors and co-activators. (Pozzo-Miller 2015).

RTT, in rare cases, may also be caused by partial gene deletions or mutations in other genes such as cyclin-dependent kinase-like 5 (CDKL5), Forkhead box protein G1 (FOXG1), and possibly other genes that have not yet been identified.

RTT manifests with incoordination, intellectual decline, gait abnormalities, and seizures. (Weng 2011). Currently, there is no treatment for RTT.

Pridopidine

Pridopidine (4-[3-(methylsulfonyl)phenyl]-1-propyl-piperidine) (formerly known as ACR16) is a drug under development for treatment of Huntington's disease. The chemical name of pridopidine is 4-(3-(Methylsulfonyl)phenyl)-1-propylpiperidine and its Chemical Registry Number is CAS 346688-38-8 (CSID:7971505 2016). The Chemical Registry number of pridopidine hydrochloride is 882737-42-0 (CSID:25948790 2016).

Pridopidine has been shown to modulate motor activity by either suppressing hyperactivity or enhancing hypoactivity. The neuroprotective properties of pridopidine are suggested to be attributed to its high affinity to the Sigma-1 receptor (S1R, binding IC50 ~ 100nM), while the motor activity of pridopidine may be mediated primarily by its low-affinity, antagonistic activity at the dopamine D2 receptor (D2R) (binding IC50 ~ 10$\mu$M) (Ponten 2010). Pridopidine shows low-affinity binding to additional receptors in the micromolar range.

The S1R is an endoplasmic reticulum (ER) chaperone protein which is implicated in cellular differentiation, neuroplasticity, neuroprotection and cognitive function in the brain. Recently, transcriptomic analysis of rat striatum showed that pridopidine treatment activates expression of the BDNF, dopamine receptor 1 (D1R), glucocorticoid receptor (GR), and the serinethreonine kinase protein kinase B (Akt)/phosphoinositide 3-kinase (PI3K) pathways, known to promote neuronal plasticity and survival and to be impaired in HD. Moreover, pridopidine gene expression profile showed a reversed pattern of the HD disease gene expression profile in a Q175 knock-in (Q175 KI) HD mouse model (Geva 2016). Pridopidine also enhances secretion of the neuroprotective brain-derived neurotrophic factor (BDNF) in a neuroblastoma cell line, in a S1R-dependent manner (Geva 2016).

WOI 2015/112601 A1 discloses a modified oral solid dosage form of pridopidine for use in treating disorders including Rett syndrome.

WO 2013/086425 A1 discloses the hydrobromide salt of pridopidine for use in treating disorders including Rett syndrome.

WO 01/46145 A1 discloses pridopidine for use in treating disorders including Rett syndrome. The three documents mentioned above do not contain any data on Rett syndrome and are silent on, the specific RTT symptoms claimed in the present application.

**SUMMARY OF THE INVENTION**

The invention is defined by the claims. Any subject-matter falling outside the scope of the claims is provided for information purposes only. Any references in the description to methods of treatment refer to the compounds and pharmaceutical compositions of the present invention for use in a method for treatment of the human or animal body by therapy.

The present invention provides a composition comprising pridopidine or its pharmaceutically acceptable salt for use in treating a subject afflicted with Rett syndrome (RTT), wherein the composition treats the subject by delaying the onset, preventing worsening, delaying worsening or improving at least one RTT symptom in the subject, wherein the RTT symptom is abnormal gait, ataxia, impaired gait initiation, a delay in acquiring purposeful hand skills or a partial or complete loss of acquired purposeful hand skills, abnormal hand movement, startle response, delayed crawling and/or walking, or decreased ability to crawl and/or walk.

In an embodiment, the pridopidine salt is pridopidine hydrochloride, hydrobromide, nitrate, perchlorate, phosphate, sulphate, formate, acetate, aconate, ascorbate, benzenesulphonate, benzoate, cinnamate, citrate, embonate, enantate, fumarate, glutamate, glycolate, lactate, maleate, malonate, mandelate, methanesulphonate, naphthalene-2-sulphonate, phthalate, salicylate, sorbate, stearate, succinate, tartrate or toluene-p-sulphonate salt.

In an embodiment, the pridopidine is administered orally, nasally, inhaled, by subcutaneous injection, or through an intravenous, intraperitoneal, intramuscular, intranasal, buccal, vaginal, rectal, intraocular, intrathecal, topical or intradermal route.

In an embodiment, the composition is administered in the form of an aerosol, an inhalable powder, an injectable, a liquid, a gel, a solid, a capsule or a tablet.

In an embodiment, the pridopidine is administered once daily or twice daily.

In an embodiment, the amount of pridopidine administered is 10 mg/day-315 mg/day; or wherein the amount of pridopidine administered in a dose is 10 mg, 22.5 mg, 45 mg, 67.5 mg, 90 mg, 100 mg, 112.5 mg, 125 mg, 135 mg, 150 mg, 180 mg, 200 mg, 250 mg, or 315 mg; or wherein the amount of pridopidine administered in a dose is 10mg-45 mg.

In an embodiment, the abnormal hand movement is wringing, squeezing, clapping, washing, tapping, rubbing, and/or repeatedly bringing hands to mouth.

In an embodiment, the pridopidine improves the symptom by at least 20%, at least 30%, at least 50%, at least 80%, or 100%.

In an embodiment, the pridopidine treats the subject by improving or maintaining the subject's ability to perform activities of daily living, perform domestic chores, manage finances, perform an occupation, and/or wherein the pridopidine treats the subject by reducing the level of nursing care needed by the subject.

In an embodiment, the pridopidine increases or maintains the BDNF serum level in the subject and/or increases the BDNF brain levels in the subject.

In an embodiment, the subject has a mutation in at least one of the methyl CpG binding protein 2 (MeCP2) gene, the cyclin-dependent kinase-like 5 (CDKL5) gene or the Forkhead box protein G1 (FOXG1) gene.

## DESCRIPTION OF THE FIGURES

**Figure 1:** Difference in feature values and feature ranks (curve covered with outlined with squares). Relative difference (%) between feature values in two different sets is calculated and plotted in the order corresponding to feature ranks together with their ranks varying from 0 to 100%.

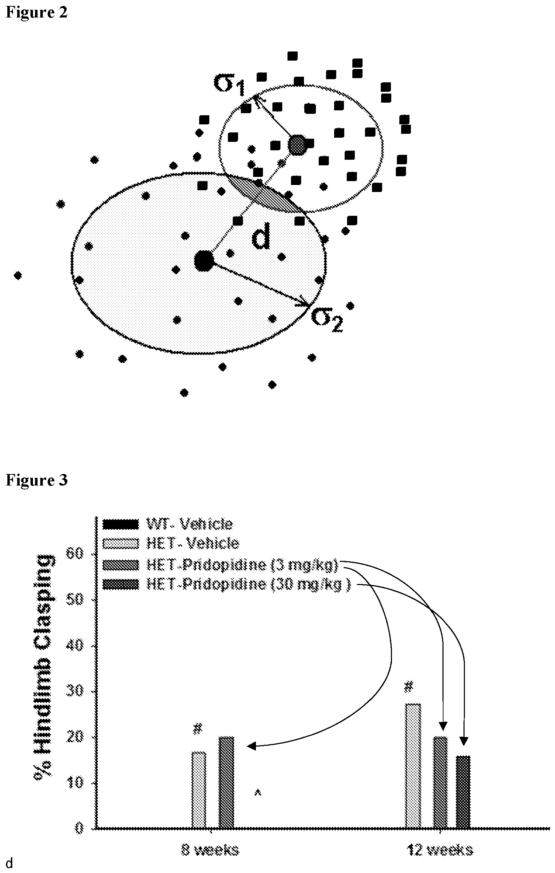

**Figure 2:** Visualization of binary discrimination in the ranked de-correlated feature space. The two highest ranked decorrelated features are chosen to form the 2D coordinate plane for visualization purposes. Each circle or square represents a mouse. Mice from the control group are shown as circles and mice from the disease group are shown as squares. The other convenient (from a scale perspective) but equivalent measure derived from the cloud overlap is discrimination probability = 1-overlap, which measures how reliably a classifier can be trained to discriminate between groups A and B above the chance level, zero corresponding to 100% overlap and no ability to distinguish the two groups above the chance level, whereas 100% meaning error free discrimination.

**Figure 3:** Percentage of mice showing hindlimb clasping (WT and pridopidine 30 mg/kg showed no clasping) at 8 weeks. [#]$p < 0.05$ compared to WT, [^]$p < 0.06$ compared to HET-vehicle group.

**Figure 4:** Latency to fall off the rotarod. Data are expressed as mean $\pm$ SEM. [#]$p < 0.05$ compared to WT-vehicle group.

**Figure 5:** Speed of Rotarod at fall. Data are expressed as mean $\pm$ SEM. [#]$p < 0.05$ compared to WT-vehicle group.

**Figure 6:** Mean startle response. Data are expressed as mean $\pm$ SEM. [#]$p < 0.05$ compared to WT-vehicle group. [*]$p < 0.05$ compared to HET-vehicle group.

**Figure 7:** Body weight of all mice throughout treatment. Data are presented as mean $\pm$ SEM. [###]$p < 0.001$, [##]$p < 0.01$, [#]$p < 0.05$ comparing HET-vehicle to WT-vehicle group; [***]$p < 0.001$ comparing HET-vehicle to HET-Pridopidine groups.

**Figures 8A-8B:** (8A) Summary of recovery analysis of gait features in MeCP2 (BIRD) mice by Pridopidine (3 mg/kg) at 8 weeks of age. (8B) Summary of recovery analysis of gait features in BIRD mice by Pridopidine (30 mg/kg) at 8 weeks of age.

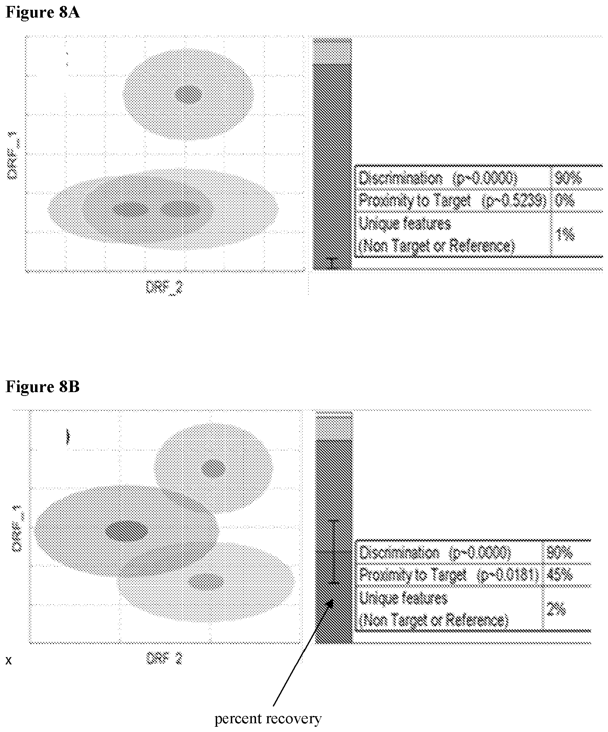

The cloud graphs are used to visualize WT (upper cloud), HET mice (lower right-most cloud), and HET + treatment (lower-left most cloud) relationship in the optimal discrimination feature space. The bar graphs show the percent recovery (darkest color) of the various treatments. Figure 8A no recovery, figure 8B 55% recovery.

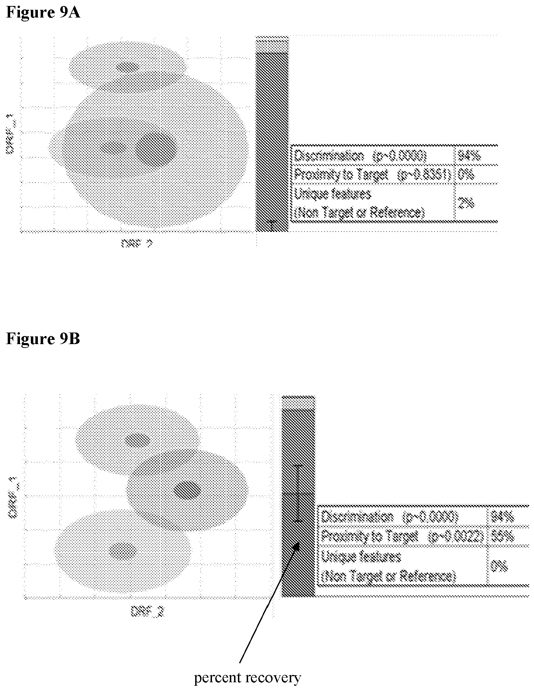

**Figures 9A-9B:** (9A) Summary of recovery analysis of gait features in BIRD mice by Pridopidine (3 mg/kg) at 12 weeks of age. (9B) Summary of recovery analysis of gait features in BIRD mice by Pridopidine (30 mg/kg) at 12 weeks of age.

The cloud graphs are used to visualize WT (upper cloud), HET mice (lower left-most cloud), and HET + treatment (lower-right most cloud) relationship in the optimal discrimination feature space. The bar graphs show the percent recovery (darkest color) of the various treatments. Figure 9A no recovery, figure 9B 55% recovery.

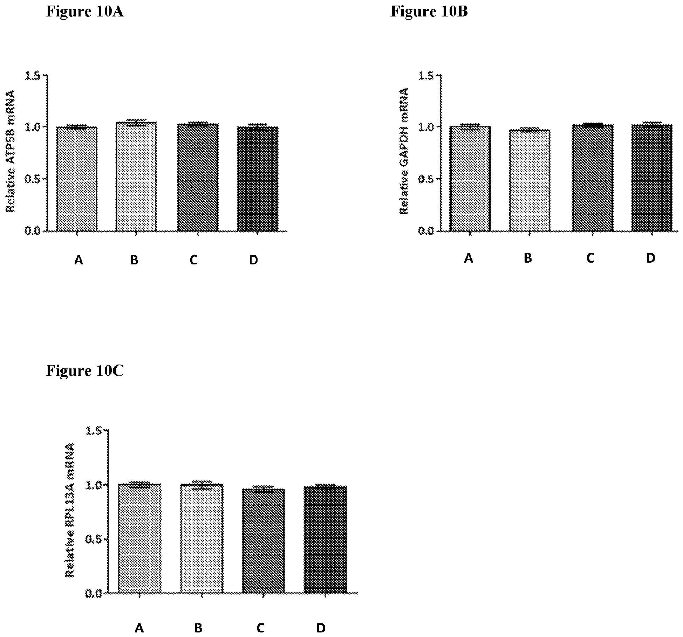

In Figures 10A-10D, 11A-11B, 12A-12B, 13A-13B and 14A-14B : Column A represents vehicle treated MeCP2 wt mice, column B represents vehicle treated MeCP2 HET mice, column C represents pridopidine treated MeCP2 mice (3mg/kg) and column D represents pridopidine treated MeCP2 mice (30mg/kg).

**Figures 10A-10C:** Relative mRNA expression of whole brain housekeeping genes: ATP5B (10A), GAPDH (10B) and RPL13A (10C); each normalized to the geometric means of the other two genes.

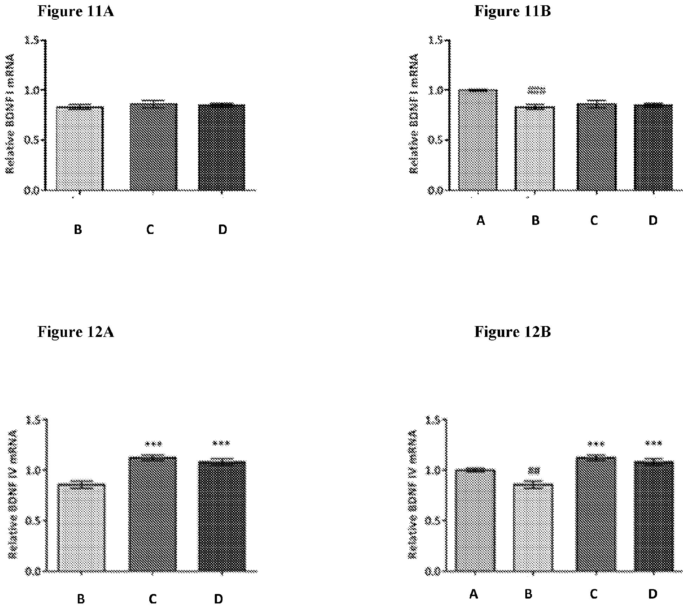

**Figures 11A-11B:** Relative mRNA expression of BDNF I in whole brain: Drug efficacy in MeCP2 (Rett) mouse model (11A), Extent of BDNF I transcript rescue as compared to MeCP2_WT, vehicle treated group (11B).

**Figures 12A-12B:** Relative mRNA expression of BDNF IV in whole brain: Drug efficacy in MeCP2 (Rett) mouse model (12A), Extent of BDNF IV transcript rescue as compared to MeCP2_WT, vehicle treated group (12B).

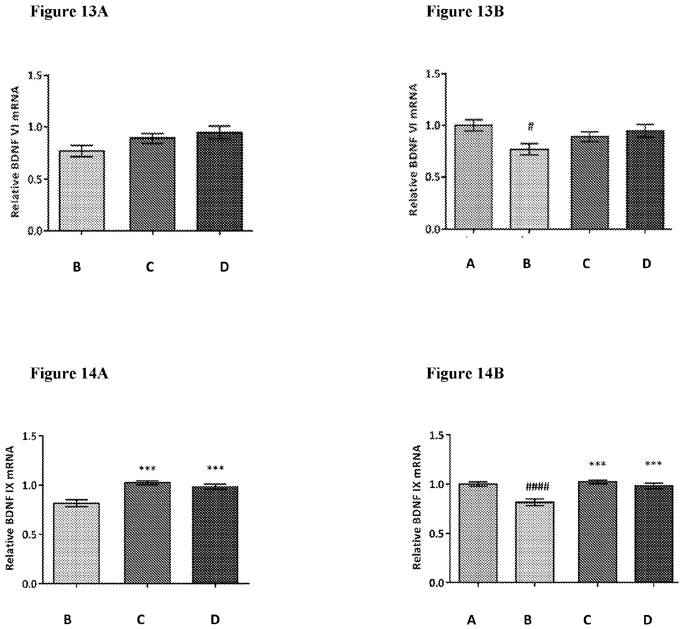

**Figures 13A-13B:** Relative mRNA expression of BDNF VI in whole brain: Drug efficacy in MeCP2 mouse model (13A), Extent of BDNF VI transcript rescue as compared to MeCP2_WT, vehicle treated group (13B).

**Figures 14A-14B:** Relative mRNA expression of BDNF IX (full length) in whole brain: Drug efficacy in MeCP2 mouse model (14A), Extent of BDNF IX transcript rescue as compared to MeCP2_WT, vehicle treated group (14B).

## DETAILED DESCRIPTION OF THE INVENTION

The present invention provides a composition comprising pridopidine or its pharmaceutically acceptable salt for use in treating a subject afflicted with Rett syndrome (RTT), wherein the composition treats the subject by delaying the onset, preventing worsening, delaying worsening or improving at least one RTT symptom in the subject, wherein the RTT symptom is abnormal gait, ataxia, impaired gait initiation, a delay in acquiring purposeful hand skills or a partial or complete loss of acquired purposeful hand skills, abnormal hand movement, startle response, delayed crawling and/or walking, or decreased ability to crawl and/or walk.

In one embodiment, the subject is a human patient. In one embodiment, the human patient is female.

In one embodiment, the subject has a mutation in the methyl CpG binding protein 2 (MECP2) gene. In one embodiment, the subject has a mutation in the cyclin-dependent kinase-like 5 (CDKL5) gene. In one embodiment, subject has a mutation in the Forkhead box protein G1 (FOXG1) gene.

In one embodiment, the pridopidine is pridopidine hydrochloride.

In one embodiment, the pridopidine is administered orally, nasally, inhaled, by subcutaneous injection, or through an intravenous, intraperitoneal, intramuscular, intranasal, buccal, vaginal, rectal, intraocular, intrathecal, topical or intradermal route. In one embodiment, the pridopidine is administered orally.

In one embodiment, the pridopidine is administered in the form of an aerosol, an inhalable powder, an injectable, a liquid, a gel, a solid, a capsule or a tablet.

In one embodiment, the pridopidine is administered periodically.

In one embodiment, the pridopidine is administered daily. In one embodiment, the pridopidine is administered once daily. In another embodiment, the pridopidine is administered more often than once daily. In one embodiment, the pridopidine is administered twice daily.

In one embodiment, the amount of pridopidine administered is 10 mg/day-315 mg/day. In one embodiment, the amount of pridopidine administered is 90 mg/day-315 mg/day. In one embodiment, the amount of pridopidine administered is 90 mg/day-225 mg/day. In one embodiment, the amount of pridopidine administered is 180 mg/day-225 mg/day. In another embodiment, the amount of pridopidine administered is 20 mg/day, 22.5 mg/day, 45 mg/day, 67.5 mg/day, 90 mg/day, 100 mg/day, 112.5 mg/day, 125 mg/day, 135 mg/day, 150 mg/day, 180 mg/day, 200 mg/day, 225 mg/day, 250 mg/day, or 315 mg/day. In an embodiment, the amount of pridopidine administered is 45 mg/day. In an embodiment, the amount of pridopidine administered is 90 mg/day. In an embodiment, the amount of pridopidine administered is 180 mg/day. In an embodiment, the amount of pridopidine administered is 225 mg/day.

In one embodiment, the amount of pridopidine is administered in one dose per day. In one embodiment, the amount of pridopidine is administered in two doses per day.

In one embodiment, the amount of pridopidine administered in a dose is 10 mg, 22.5 mg, 45 mg, 67.5 mg, 90 mg, 100 mg, 112.5 mg, 125 mg. 135 mg, 150 mg, 180 mg, 200 mg, 250 mg, or 315 mg. In an embodiment, the amount of pridopidine administered in a dose is 45 mg. In an embodiment, the amount of pridopidine administered in a dose is 10-45 mg.

In one embodiment, the amount of pridopidine is administered in two doses per day at an amount of 45 mg per dose.

In one embodiment, the pridopidine is first administered within 1 day after birth of the subject. In one embodiment, the pridopidine is first administered within 1 week after birth of the subject. In one embodiment, the pridopidine is first administered within 1 month after birth of the subject. In one embodiment, the pridopidine is first administered within 3 months after birth of the subject. In one embodiment, the pridopidine is first administered within 6 months after birth of the subject. In one embodiment, the pridopidine is first administered within 9 months after birth of the subject. In one embodiment, the pridopidine is first administered within 12 months after birth of the subject. In one embodiment, the pridopidine is first administered within 18 months after birth of the subject. In one embodiment, the pridopidine is first administered within 3 years after birth of the subject. In one embodiment, the pridopidine is first administered within 5 years after birth of the subject. In one embodiment, the pridopidine is first administered within 10 years after birth of the subject. In one embodiment, the pridopidine is first administered within 15 years after birth of the subject. In one embodiment, the pridopidine is first administered within 20 years after birth of the subject. In one embodiment, the pridopidine is first administered within 25 years after birth of the subject. In one embodiment, the pridopidine is first administered within 30 years after birth of the subject. In one embodiment, the pridopidine is first administered 30 years or more after birth of the subject.

In one embodiment, the periodic administration of pridopidine continues for at least 3 days, at least 30 days, at least 42 days, at least 8 weeks, at least 12 weeks, at least 24 weeks, at least 6 months, at least 1 year, at least 2 years, at least 5 years, at least 10 years, at least 15 years, at least 20 years, at least 25 years, or 30 years or more.

In one embodiment, the abnormal hand movement is wringing, squeezing, clapping, washing, tapping, rubbing, and/or repeatedly bringing hands to mouth.

In one embodiment, the pridopidine improves the symptom by at least 20%. In one embodiment, the pridopidine improves the symptom by at least 30%. In one embodiment, the pridopidine improves the symptom by at least 50%. In one embodiment, the pridopidine improves the symptom by at least 80%. In one embodiment, the pridopidine improves the symptom by 100%. In one embodiment, the pridopidine treats the subject by improving the subject's ability to perform activities of daily living, perform domestic chores, manage finances, and/or perform an occupation. In one embodiment, the pridopidine treats the subject by reducing the level of nursing care needed by the subject.

In one embodiment, the pridopidine treats the subject by maintaining the subject's ability to perform activities of daily living, perform domestic chores, manage finances, and/or perform an occupation.

In one embodiment, the pridopidine is effective to increase the BDNF serum level in the subject. In one embodiment, the pridopidine is effective to increase the BDNF levels in the brain of the subject. In one embodiment, the pridopidine is effective to maintain the BDNF serum level in the subject.

In an embodiment, the composition is a pharmaceutical composition, for example in unit dosage form.

In one embodiment, the amount of pridopidine is 10 mg-315 mg. In one embodiment, the amount of pridopidine is 90 mg-315 mg. In one embodiment, the amount of pridopidine is 90 mg-225 mg. In another embodiment, the amount of pridopidine is 22.5 mg, 45 mg. 67.5 mg, 90 mg, 100 mg, 112.5 mg, 125 mg, 135 mg. 150 mg, 180 mg, 200 mg, 225 mg, 250 mg, or 315 mg. In an embodiment, the amount of pridopidine is 45 mg. In an embodiment, the amount of pridopidine is 90 mg. In an embodiment, the amount of pridopidine is 180 mg. In an embodiment, the amount of pridopidine is 225 mg.

In an embodiment, the composition may be provided in the form of a package comprising:

a) a pharmaceutical composition comprising an amount of pridopidine and a pharmaceutically acceptable carrier; and
b) instructions for use of the pharmaceutical composition to treat a subject afflicted with RTT.

In an embodiment, the composition may be provided in the form of a therapeutic package for dispensing to, or for use in dispensing to, a subject afflicted with RTT, which comprises:

a) one or more unit doses, each such unit dose comprising an amount of pridopidine effective to treat the subject afflicted with RTT, and
b) a finished pharmaceutical container therefor, said container containing said unit dose or unit doses, said container further containing or comprising labeling directing the use of said package in treating the subject.

Terms

As used herein, and unless stated otherwise, each of the following terms shall have the definition set forth below.

As used herein, "pridopidine" means pridopidine base or a pharmaceutically acceptable salt thereof, as well as derivatives, for example deuterium-enriched pridopidine and salts. Examples of deuterium-enriched pridopidine and salts and their methods of preparation may be found in U.S. Patent Application Publication Nos. 2013-0197031, 2016-0166559 and 2016-0095847.

"Deuterium-enriched" means that the abundance of deuterium at any relevant site of the compound is more than the abundance of deuterium naturally occurring at that site in an amount of the compound. The naturally occurring distribution of deuterium is about 0.0156%. Thus, in a "deuterium-enriched" compound, the abundance of deuterium at any of its relevant sites is more than 0.0156% and can range from more than 0.0156% to 100%. Deuterium-enriched compounds may be obtained by exchanging hydrogen with deuterium or synthesizing the compound with deuterium-enriched starting materials.

The active compound for use according to the invention may be provided in any form suitable for the intended administration. Suitable forms include pharmaceutically acceptable salts of the compound of the invention.

A "salt thereof' is a salt of the instant compound which has been modified by making acid or base salts of the compound. The term "pharmaceutically acceptable salt" in this respect, refers to the relatively non-toxic, inorganic and organic acid or base addition salts of compound of the present invention suitable for pharmaceutical use. Pharmaceutically acceptable salts may be formed by procedures well known and described in the art. One means of preparing such a salt is by treating a compound of the present invention with an inorganic base.

Examples of acid addition salts of the compound of the present invention include the hydrochloride, the hydrobromide, the nitrate, the perchlorate, the phosphate, the sulphate, the formate, the acetate, the aconate, the ascorbate, the benzenesulphonate, the benzoate, the cinnamate, the citrate, the embonate, the enantate, the fumarate, the glutamate, the glycolate, the lactate, the maleate, the malonate, the mandelate, the methanesulphonate, the naphthalene-2-sulphonate, the phthalate, the salicylate, the sorbate, the stearate, the succinate, the tartrate, the toluene-p-sulphonate, and the like. In certain embodiments, pridopidine is a pharmaceutically acceptable salt, such as the HCl salt or tartrate salt. Preferably, in any embodiments of the invention as described herein, the pridopidine is in the form of its hydrochloride salt.

As used herein, an "amount" or "dose" of pridopidine as measured in milligrams refers to the milligrams of pridopidine (4-[3-(methylsulfonyl)phenyl]-1-propyl-piperidine) present in a preparation, regardless of the form of the preparation. For example, a unit dose containing "90 mg pridopidine" means the amount of pridopidine in a preparation is 90 mg, regardless of the form of the preparation. Thus, when in the form of a salt, e.g. pridopidine hydrochloride, the weight of the salt form necessary to provide a dose of 90 mg pridopidine would be greater than 90 mg due to the presence of the salt.

As used herein, a "unit dose", "unit doses" and "unit dosage form(s)" mean a single drug administration entity/entities. A "unit dose", "unit doses" and "unit dosage form(s)" can be prepared for oral dosage forms, such as tablets, capsules, pills, powders, and granules.

As used herein, "about" in the context of a numerical value or range means 90-110% of the numerical value or range recited or claimed.

"Administering to the subject" or "administering to the (human) patient" means the giving of, dispensing of, or application of medicines, drugs, or remedies to a subject/patient to delay, relieve, cure, or reduce the symptoms associated with a condition, e.g., a pathological condition. Oral administration is one way of administering the instant compounds to the subject.

A compound according to the subject invention may be administered in the base form or in the form of pharmaceutically acceptable salts, preferably in a pharmaceutical composition together with one or more adjuvants, excipients, carriers, buffers, diluents, and/or other customary pharmaceutical auxiliaries.

A "pharmaceutically acceptable carrier" refers to a carrier or excipient that is suitable for use with humans and/or animals without undue adverse side effects (such as toxicity, irritation, and allergic response) commensurate with a reasonable benefit/risk ratio. It can be a pharmaceutically acceptable solvent, suspending agent or vehicle, for delivering the instant compound to the subject.

The administration can be periodic administration. As used herein, "periodic administration" means repeated/recurrent administration separated by a period of time. The period of time between administrations is preferably consistent from time to time. Periodic administration can include administration, e.g., once daily, twice daily, three times daily, four times daily, weekly, twice weekly, three times weekly, four times weekly and so on, etc.

"Treat" or "treating" as used herein encompasses alleviating, lessening, reducing the severity of, eliminating or substantially eliminating, or ameliorating a physical, mental or emotional limitation in a subject afflicted with RTT.

Treating also refers to delaying or prevention of symptoms or reduction of deficits associated with a disease.

As used herein, "effective" as in an amount effective to achieve an end means the quantity of a component that is sufficient to yield an indicated therapeutic response without undue adverse side effects (such as toxicity, irritation, or allergic response) commensurate with a reasonable benefit/risk ratio when used in the manner of this disclosure. For example, an amount effective to treat a symptom of RTT. The specific effective amount varies with such factors as the particular condition being treated, the physical condition of the patient, the type of mammal being treated, the duration of the treatment, the nature of concurrent therapy (if any), and the specific formulations employed and the structure of the compounds or its derivatives.

It is understood that where a parameter range is provided, all integers within that range, and tenths thereof, are also provided by the invention. For example, "22 mg - 300.0 mg" includes 22.0 mg, 22.1 mg, 22.2 mg, 22.3 mg, 22.4 mg, etc. up to 300.0 mg inclusive.

This invention will be better understood by reference to the Experimental Details which follow, but those skilled in the art will readily appreciate that the specific experiments detailed are only illustrative of the invention as described more fully in the claims which follow thereafter.

## EXPERIMENTAL DETAILS

### Example 1: Evaluation Of The Efficacy of Pridopidine In The MeCP2 Mouse Model Of Rett Syndrome

The goal of this study was to assess the effects of pridopidine in the female MeCP2 (BIRD) mouse model of RTT (Guy 2001).

### Materials:

Pridopidine (3 and 30 mg/kg) was administered orally twice daily (6 hours between dosing) at a dose volume of 10 ml/kg. On test days, pridopidine was administered 30 minutes prior to test.

Dosing commenced when mice were ~5.5 weeks of age and continued through the end of behavioral testing. Behavioral testing was done at 8 and 12 weeks of age.

Female MeCP2 (MeCP1_HET, HET) mice and wild type (MeCP2 WT, WT) littermates were received in two cohorts at ~4.5 weeks of age from Jackson Laboratories. They were assigned unique identification numbers and group-housed in opti-MICE cages (Animal Care Systems, CO). All animals were examined and weighed prior to initiation and throughout the study to assure adequate health and suitability and to minimize nonspecific stress associated with manipulation. During the course of the study, 12/12 light/dark cycle was maintained. The room temperature was maintained between 20 and 23°C with a relative humidity maintained around 50%. Chow and water was provided ad libitum for the duration of the study. The tests were performed during the animal's light cycle phase.

### Methods:

**Treatment Groups:**

- WT mice - vehicle (subcutaneous once weekly, saline), n=24 (n=20 cohort 1; n=4 cohort 2)
- HET MeCP2 mice - vehicle (subcutaneous once weekly, saline), n=24 (n=20 cohort 1; n=4 cohort 2)
- HET MeCP2 mice - Pridopidine (3 mg/kg; orally twice daily), n=20 (cohort 2)
- HET MeCP2 mice - Pridopidine (30 mg/kg; orally twice daily), n=20 (cohort 1)

**Body Weights:**

- Body weight was assessed twice weekly

**Behavioral Tests:**

*(1) Gait Analysis using NeuroCube® System*

The NeuroCube® system is a platform that employs computer vision to detect changes in gait geometry and gait dynamics in rodent models of neurological disorders, pain & neuropathies. This platform is unique for gait testing for the following reasons:

- It is completely automated and thus removes any bias or subjectivity
- This system captures both gait geometry and gait dynamics (stance, swing, propulsion, etc.)

Mice were placed in the NeuroCube for a 5 min test. The most dominant of the features collected that define the disease phenotype (symptom descriptors) was identified and ranked. Complex bioinformatic algorithms were employed to calculate the discrimination probability between the WT and the BIRD mice and detect a test compound's ability to reverse the disease phenotype. Discriminations between mutant and wild type was calculated as well as the recovery of disease features in HET mice treated with the test compound.

*(2) Clasping*

Clasping is used to assess muscular strength in limb muscles. Mice were held by the tail and gently lifted until the front paws just lift off the counter surface. The experimenter observed the legs and determined clasping or splaying of limbs. After testing, animals were placed back into the test or home cage. Percent clasping of the hindlimbs was determined and reported.

*(3) Rotarod*

Mice were taken to the experimental room and placed on the rotarod apparatus. The rod rotates at a constant or variable and accelerating speed of 4 rpm. Once a mouse lost its balance and fell onto an underlying platform the timer automatically stopped. Paper towels or diaper pads were used to cushion the fall. The latency time in sec that it took for an animal to fall or the endurance time for each mouse was automatically recorded by the equipment. Mice were exposed to the apparatus for 5 min training each time tested at a constant speed and placed back on the rod after each fall. After a rest period of at least 1 hr animals were placed back on the rotarod apparatus for testing. Once all animals in a test session were loaded on the rod, the rotarod apparatus was placed on accelerating speed (0-40 rpm) over 5 min and the time until the first fall was recorded. Fall speed and latency to fall were recorded.

*(4) Startle Response/Prepulse Inhibition (PPI)*

The acoustic startle measures an unconditioned reflex response to external auditory stimulation. Prepulse inhibition (PPI) consisting of an inhibited startle response (reduction in amplitude) to an auditory stimulation following the presentation of a weak auditory stimulus or prepulse, has been used as a tool for the assessment of deficiencies in sensory-motor gating, such as those seen in schizophrenia. This is an optional test that would only be performed on those animals that do not exhibit audiogenic seizures.

Mice were placed in the PPI chambers (Med Associates) for a 5 min session of white noise (70 dB) habituation. After the acclimation period the test session automatically started. The session started with a habituation block of 6 presentations of the startle stimulus alone, followed by 10 PPI blocks of 6 different types of trials.

Trial types were: null (no stimuli), startle (120 dB), startle plus prepulse (4, 8 and 12 dB over background noise i.e. 74, 78 or 82 dB) and prepulse alone (82 dB). Trial types were presented at random within each block. Each trial started with a 50 ms null period during which baseline movements were recorded. There was a subsequent 20 ms period during which prepulse stimuli were presented and responses to the prepulse were measured. After further 100 ms the startle stimuli were presented for 40 ms and responses recorded for 100 ms from startle onset. Responses were sampled every millisecond. The inter-trial interval was variable with an average of 15 s (range from 10 to 20 s).

In startle alone trials the basic auditory startle was measured and in prepulse plus startle trials the amount of inhibition of the normal startle was determined and expressed as a percentage of the basic startle response (from startle alone trials), excluding the startle response of the first habituation block.

**Brain Collections:**

After all behavioral testing was completed brain samples were collected 60 minutes after dosing with pridopidine. Mice were euthanized via cervical dislocation and decapitated. From 10 mice/treatment group, whole brains were collected, weighed, and then frozen on dry ice. Samples were stored at - 80°C until analysis of brain-derived neurotrophic factor (BDNF).

From the remaining 10 brains, whole brains were collected and then cut sagital. From the left hemisphere striatum, PFC, and hippocampus were dissected and placed in RNAlater. From the right hemisphere, striatum, PFC, and hippocampus were collected, snap frozen on dry ice, and stored at - 80°C until shipment to Sponsor's Designated Laboratory for protein analysis.

**BDNF Analysis:**

*Total RNA Extraction:*

Tissues (whole brain) were homogenized 2 × 1min at 25 Hz in 750$\mu$L of QIAzol Lysis Reagent (Cat # 79306, Qiagen, Valencia, CA) with TissueLyser (Qiagen, Valencia, CA) and 5mm stainless steel beads (Cat # 69989, Qiagen, Valencia, CA). Once tissues were disrupted, samples were allowed to incubate at room temperate for 5 minutes.

For RNA extraction, manufacturer protocol for RNeasy 96 Universal Tissue Kit (Cat # 74881, Qiagen, Valencia, CA) for RNA isolation was followed. Briefly, 150$\mu$L of Chloroform (Cat # C2432, Sigma-Aldrich, St. Louis, MO) was added and samples were shaken vigorously for 15 seconds followed by 3-minute incubation at room temperature. The aqueous phase was separated from the organic phase by centrifugation at 6,000 x g (Beckman Coulter Avanti J-30I), 4°C for 15 minutes. The aqueous phase was then transferred to a new 96-well block and total RNA was precipitated with equal volume of 70% ethanol. Content was transferred to an RNeasy 96-well plate, followed by centrifuge at 6,000-x g (Beckman Coulter Avanti J-30I), at room temperate for 4 minutes. Total RNA bound to column membranes was treated with RNase-Free DNase set (Cat # 79254, Qiagen, Valencia, CA) for 30 minutes, followed by 3 washing steps with RW1 and RPE buffers (provided with RNeasy 96 Universal Tissue Kit). RNA was eluted with RNase-Free water.

*Total RNA Quantification and Reverse Transcription:*

Samples were quantified using NanoDrop 8000 (Thermo Scientific). One microgram of total RNA was reverse transcribed into cDNA with 3.2$\mu$g random hexamers (Cat # 11034731001, Roche Applied Science, Indianapolis, IN), 1mM each dNTP (Cat # 11814362001), Roche Applied Science, Indianapolis, IN), 20U Protector RNase Inhibitor (Cat # 03335402001, Roche Applied Science, Indianapolis, IN), IX Transcriptor Reverse Transcription reaction buffer and 10U Transcriptor Reverse Transcriptase (Cat # 03531287001, Roche Applied Science, Indianapolis, IN) in 20$\mu$L total volume.

Up to three independent RT reactions were performed for each RNA sample. The reactions were allowed to proceed at room temperature for 10 minutes, 55°C for 30 minutes, and then inactivated at 85°C for 5 minutes in GeneAmp PCR Systems 9700 thermal cycler (Applied Biosystems, Foster City, CA). cDNA samples were diluted 10 folds with RNase-Free water for qPCR analysis.

*Quantitative PCR (qPCR):*

For all reactions utilizing Universal Probe Library Probes, 5$\mu$l of the diluted cDNA was amplified with 12.5$\mu$L 2x FastStart Universal Probe Master Rox (Cat # 04914058001, Roche Applied Science, Indianapolis, IN), 0.5$\mu$L Universal Probe Library Probe (Roche Applied Science, Indianapolis, IN), 200nM of gene specific primer- HPLC purified (Sigma-Aldrich, St. Louis, MO) in 25 $\mu$L reaction volume. The reactions were run on the ABI 7900HT Sequence Detection System (Applied Biosystems, Foster City, CA). qCPR conditions were 95°C for 10 minutes for activation of FastStart Taq DNA Polymerase followed by 40 cycles of 95°C for 15 seconds and 60°C for 1 minute. For primers and Universal Probe Library used for qPCR please refer to Table 1 below.

Table 1: qPCR and primers/probe information

| Mouse Gene ID | 5' Primer Sequence | 3' Primer Sequence | Universal Probe Library # | Tissue | PCR Efficien cy |
|---|---|---|---|---|---|
| ATP5 B | GGCACAATGCAG GAAAGG (SEQ ID NO: 1) | TCAGCAGGCACATAGAT AGCC (SEQ ID NO: 2) | 77 | Brain | 1.89 |
| RPL1 3A | TTGTGGCCAAGC AGGTACT (SEQ ID NO: 3) | GTTGATGCCTTCACAGC GTA (SEQ ID NO: 4) | 77 | Brain | 1.91 |
| GAPD H | CAATGTGTCCGTC GTGGATCT (SEQ ID NO: 5) | GTCCTCAGTGTAGCCCA AGATG (SEQ ID NO: 6) | N/A | Brain | 1.87 |
| BDNF I | AGTCTCCAGGAC AGCAAAGC (SEQ ID NO: 7) | TGCAACCGAAGTATGAA ATAACC (SEQ ID NO: 8) | 31 | Brain | 2.00 |
| BDNF IV | GCTGCCTTGATGT TTACTTTGA (SEQ ID NO: 9) | AAGGATGGTCATCACTC TTCTCA (SEQ ID NO: 10) | 31 | Brain | 2.04 |
| BDNF VI | CCGAGAGCTTTGT GTGGAC (SEQ ID NO: 11) | TCATGCAACCGAAGTAT GAAA (SEQ ID NO: 12) | 31 | Brain | 1.93 |
| BDNF IX | GCCTTTGGAGCCT CCTCTAC (SEQ ID NO: 13) | GCGGCATCCAGGTAATT TT (SEQ ID NO: 14) | 67 | Brain | 2.01 |
| qPCR Data Analysis: | | | | | |

Whole brain cDNA prepared from a pool sample of WT vehicle treated animals was used as calibrator (calibrator is diluted same as sample cDNA) to normalized plate to plate variations. See Table 1 above for PCR efficiencies of the qPCR assays used in this study.

Each cDNA sample (diluted 1:10) was assayed in triplicates and the Ct values averaged. Values that lie greater than 0.5 standard deviation of the average were discarded.

Relative quantity of the PCR product (relative to the calibrator) was calculated as follows:

$$\textit{Relative Quantity of Target gene} = \textit{(PCR EfficiencyTarget)}^{(Ct\ calibrator-Ctsample)}$$

$$\textit{Relative Quantity of Housekeeping Gene 1} = \textit{(PCR Efficiencyhousekeeping1)}^{(Ct\ calibrator-Ctsample)}$$

$$\textit{Relative Quantity of Housekeeping Gene 2} = \textit{(PCR Efficiencyhousekeeping2)}^{(Ct\ calibrator-Ctsample)}$$

$$\textit{Relative Quantity of Housekeeping Gene 3} = \textit{(PCR Efficiencyhousekeeping3)}^{(Ct\ calibrator-Ctsample)}$$

Geometric mean for the three housekeeping genes was calculated as follows:

$$\textit{Geometric mean} = \textit{(relative quantity of housekeeping gene 1 * relative quantity of housekeeping gene 2 * relative quantity of housekeeping gene 3)}^{(1/3)}$$

Relative level of target gene was calculated as follows:

$$\textit{Relative Quantity of Target gene} \div \textit{Geometric mean of housekeeping genes}$$

Relative level of target gene was then normalized to the WT vehicle group.

**Statistical Analysis:**

Data from standard tests were analyzed by genotype (t-test) and by treatment (ANOVA) followed by post-hoc comparisons where appropriate. For some measures (i.e. body weights and PPI), repeatedmeasures ANOVAs were performed. For clasping data, N-1 two-proportional tests were performed. An effect was considered significant if $p < 0.05$. All data are represented as the mean and standard error to the mean (s.e.m). Values $\pm$ 2 standard deviations from the mean were considered outliers.

*Data analysis from NeuroCube:*

The output of NeuroCube is a set of dozens of behavioral features that are submitted for analysis with machine learning techniques used in bioinformatics. Many of these features are correlated (e.g. rearing counts and supported rearing counts). Therefore, PGI forms statistically independent combinations of the original features (further referred to as de-correlated features) that discriminate between the two groups more effectively.

Each de-correlated feature extracts information from the whole cluster of the original features, so the new feature space has lower dimensionality. Next, PGI applies a proprietary feature ranking algorithm to score each feature's discrimination power (ability to separate the two groups, e.g. control and disease).

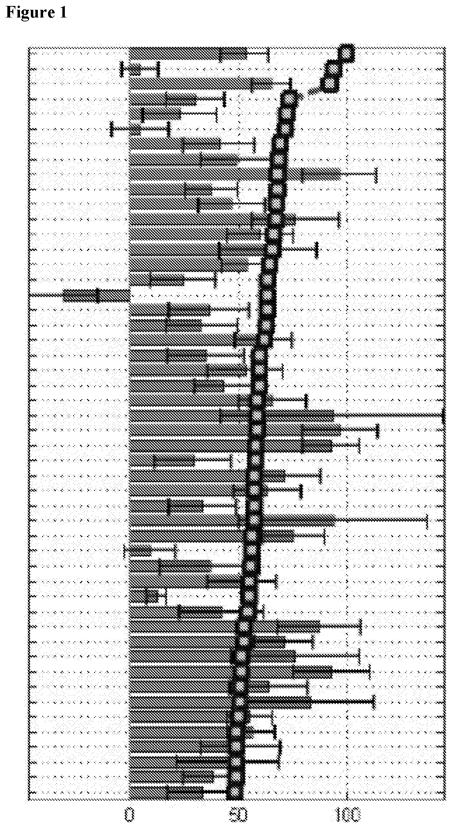

Ranking is an important part of the analyses because it weighs each feature change by its relevance: if there is a significant change in some irrelevant feature measured for a particular phenotype, the low rank of this feature will automatically reduce the effect of such change in the analyses, so there is no need to resort to the conventional "feature selection" approach and discard information buried in the less informative features. Ranking algorithm can be applied to either original or the new features to gain insight about the key control-disease differences (see **Figure 1**).

*Feature analysis: quantitative assessment of Disease Phenotype*

In the new feature space, the overlap between the "clouds" (Gaussian distributions approximating the groups of mice in the ranked de-correlated features space) serves as a quantitative measure of separability ("distinguishability") between the two groups (see **Figure 2**). For visualization purposes, each cloud was plotted with its semi-axes equal to the one standard deviation along the corresponding dimensions.

**Results:**

In figures 3-6 the black columns to the left represent vehicle treated wt animals, the light gray columns represent vehicle treated HET animals, the darker gray columns represent pridopidine treated HET animals (3 mg/kg) and the darkest gray columns to the right represent pridopidine treated HET animals (30 mg/kg).

**Behavioral Tests:**

*(1) Clasping*

The effects of pridopidine on clasping at 8 and 12 weeks are shown in **Figure 3**. Vehicle-treated HET mice showed significantly more clasping compared to the WT mice. Pridopidine (3 mg/kg BID) did not have a significant effect on this measure. However, pridopidine (30 mg/kg BID) tended to normalize this behavior (p < 0.06) at 8 weeks but this effect was not seen at 12 weeks. (At 8 weeks, columns representing the wt vehicle treated and pridopidine treated (30 mg.kg) animals are missing. At 12 weeks the column representing wt vehicle treated animals is missing).

*(2) Rotarod*

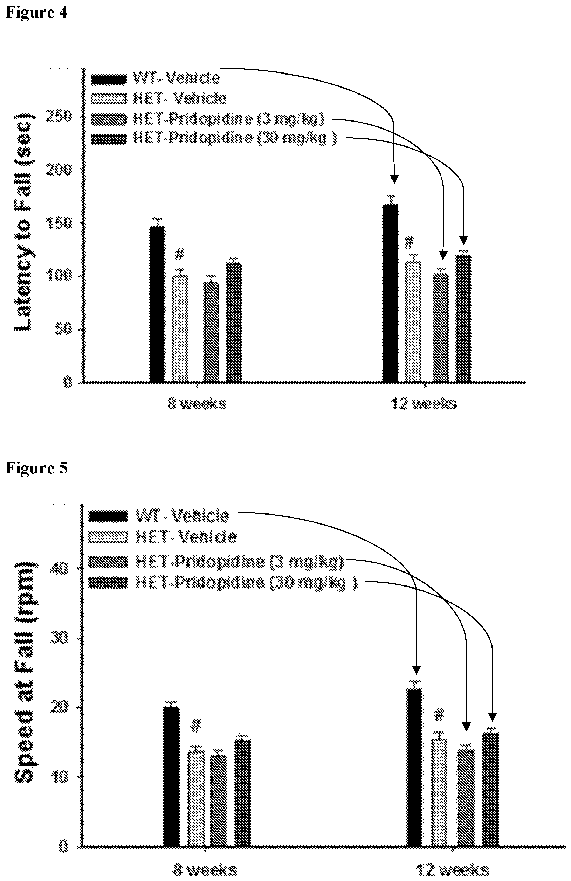

The effects of test compounds on the latency to fall and speed at fall from a rotating rod are shown in **Figures 4 and 5**, respectively. MeCP2_HET mice fell more rapidly and at slower rotating speeds compared to WT mice. There was no effect of treatment on either of these measures.

*(3) Startle Response/PPI*

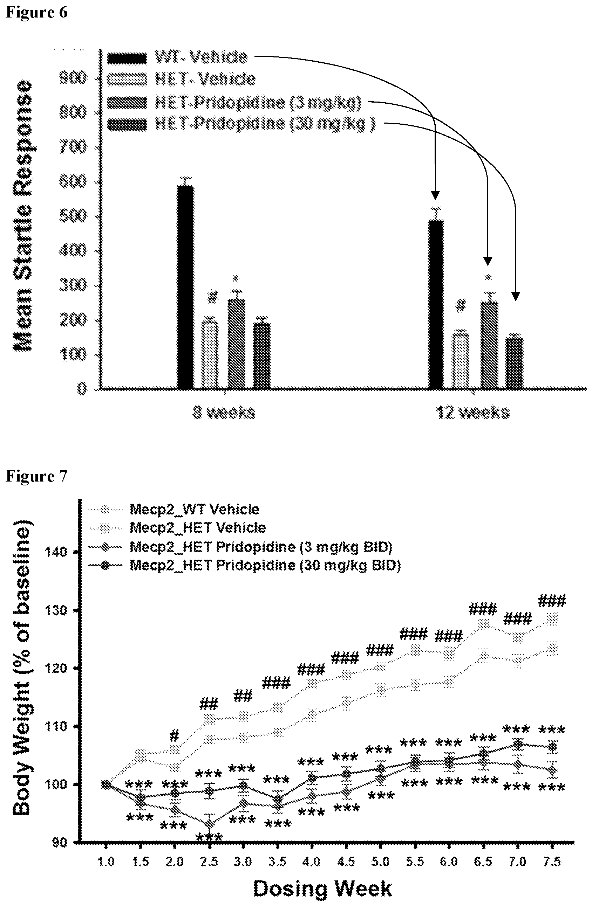

The effects of pridopidine on the startle response are shown in **Figure 6**. Vehicle-treated HET mice startled less compared to vehicle-treated WT mice. Pridopidine (30 mg/kg BID) did not have a significant effect on this measure. However, HET mice treated with pridopidine (3 mg/kg BID) showed increased startle response compared to vehicle-treated HET mice at both ages. No genotype or treatment effects were observed in the prepulse inhibition response, but this response is historically variable and not used as the primary measure for this test but collected in the course of testing.

*(4) Body Weight*

The percent change in body weights of all mice during the treatment period are shown in **Figure 7**. Repeated measures ANOVA found a significant genotype effect and genotype x time interaction. The percent increase in BW was higher in the HET mice compared to WT mice from the second week of the study until study completion.
Within HET mice, repeated measures ANOVA found a significant treatment effect. Both groups treated with pridopidine (3 mg/kg and 30 mg/kg BID) showed little weight gain from 1.5 weeks posttreatment onward compared to vehicle. In the figure, light gray circles represent vehicle treated wild type mice; light gray squares represent vehicle treated MeCP2 HET mice, dark gray diamonds represent pridopidine treated MeCP2 HET mice (3 mg/kg), and dark gray circles represent pridopidine treated MeCP2 HET mice (30 mg/kg).

*(5) NeuroCube®*

The discrimination probability between WT and HET mice at 8 and 12 weeks of age were 90% and 94%, respectively. Some of the top gait features that discriminated between WT and HET include longer stride and step length, narrower base width, and less paw intensity of WT mice compared to HET mice at 8 weeks of age. At 12 weeks of age WT mice also showed longer stride length, stride duration, and swing duration, larger paw area and less paw intensity compared to HET mice. The effects of pridopidine on gait performance at 8 weeks are shown in **Figure 8.** The effects of pridopidine on gait performance at 12 weeks are shown in **Figure 9**. Pridopidine (30 mg/kg) showed significant recovery of overall gait features at 8 weeks and 12 weeks (45% and 55 %, respectively).

Further analysis showed significant differences in several gait domains as shown in Table 2 below. The MeCP2_HET mice were significantly different from the WT control mice overall, in all gait features (except rhythmicity at 8 weeks). Week 8 data suggest that MeCP2_HET mice treated with pridopidine (3 and 30 mg/kg BID) showed efficacy on body motion at both ages. Significant effects on gait alone were also seen with pridopidine (30 mg/kg BID) at the 12 week time-point.

Table 2: Effects of pridopidine on gait at 8 and 12 weeks.

| 8 weeks | | | | |
|---|---|---|---|---|
| | *WT vehicle v. Het vehicle* | | *Recovery* | |
| **Feature** | **Discrimination Probability (%)** **p value** | | *(%) Pridopidine, 3mg/kg* **BID** | **Pridopidine 30 mg/kg** **BID** |
| Gait | 92 | = 0 | 38 | 71 |
| Paw Features | 70 | < 0.007 | 0 | 63 |
| Rhythmicity | 55 | > 0.34 | -- | -- |
| Body Motion | 79 | <0.001 | 81 | 84 |
| Paw Positioning | 87 | < 0.001 | 8 | 35 |
| 12 weeks | | | | |
| | *WT vehicle v. Het* | | *Recovery (%)* | |
| **Feature** | **Discrimination Probability (%)** **p value** | | **Pridopidine 3mg/kg BID** | **Pridopidine 30 mg/kg** **BID** |
| Gait | **94** | **= 0** | 60 | 100 |
| Paw Features | **74** | **< 0.002** | 0 | 69 |
| Rhythmicity | **70** | **< 0.007** | 41 | 10 |
| Body Motion | **83** | **= 0** | 65 | 59 |
| Paw Positioning | **87** | **< 0.001** | 35 | 52 |

**BDNF Analysis**

The effects of pridopidine on relative BDNF expression in brain samples of the WT and HET mice are shown in **Figures 10-14.**

Whole brain housekeeping genes mRNA expression levels were not changed between the different animal groups treatments examined (see Figure 10).

As compared with MeCP2_WT (vehicle), BDNF I mRNA expression was significant decreased in MeCP2_HET (vehicle) treated group. No significant changes were observed in the MeCP2_HET treated groups as compared with MeCP2_HET vehicle treated group (see Figure 11).

As compared with MeCP2_WT (vehicle), BDNF IV mRNA expression was significant decreased in MeCP2_HET (vehicle) treated group. Pridopidine treatment (3 or 30mg/kg) rescued downregulated BDNF IV mRNA in MeCP2_HET mice close to MeCP2_WT levels (see Figure 12).

As compared with MeCP2_WT (vehicle), BDNF VI mRNA expression was significant decreased in MeCP2_HET (vehicle) treated group. No significant changes were observed in the MeCP2_HET treated groups as compared with MeCP2_HET vehicle treated group (see Figure 13).

As compared with MeCP2_WT (vehicle), BDNF IX mRNA expression was significant decreased in MeCP2_HET (vehicle) treated group. Pridopidine treatment (3 or 30mg/kg) rescued downregulated BDNF IX mRNA in MeCP2_HET mice close to MeCP2_WT levels (see Figure 14).

**Conclusion**

This study evaluated the effects of chronic subcutaneous administration of pridopidine on gait, clasping, rotarod, and startle/PPI in MeCP2_HET mice.

Pridopidine (3 mg/kg BID) differed significantly from vehicle-treated mutants in gait measures. Additionally, HET mice treated with pridopidine (3 mg/kg BID) showed increased startle response compared to vehicle-treated HET mice at both testing time points. MeCP2_HET mice treated with pridopidine (30 mg/kg BID) showed significant recovery of gait features and tended to normalize clasping at 8 weeks of age.

Our previous data showed that transcripts encoding for BDNF I, IV, VI, IX isoforms, generated from alternative splicing but producing the same protein, are down regulated in the brain of 4 months old heterozygous MeCP2 knockout mice.

By comparing relative mRNA expression levels of these transcripts in the brain of vehicle treated MeCP2_HET and WT mice, previous observations were reproduced.

None of the treatments had a significant effect on BDNF I and BDNF VI mRNA expression levels in MeCP2 mice. Treatment with both doses of Pridopidine (3 and 30 mg/kg BID) fully rescued the downregulated mRNA levels of BDNF IV and BDNF IX. Positive effect of Pridopidine on expression of BDNF mRNA is consistent with improvement observed in behavioral paradigms.

**Example 2: RNA analysis of pridopidine treated MeCP2 mice**

Female MeCP2 (HET, heterozygous) mice and wild type littermates at ~4.5 weeks of age were treated with either pridopidine or vehicle. Pridopidine (3 and 30 mg/kg) was administered orally twice daily (6 hours between dosing) at a dose volume of 10 ml/kg. There were four treatment groups: 1. WT mice - vehicle, 2. HET MeCP2 mice - vehicle, 3. HET MeCP2 mice - Pridopidine (3 mg/kg; PO twice daily), 4. HET MeCP2 mice - Pridopidine (30 mg/kg; PO twice daily).

RNA was isolated from striatum, hippocampus, and cortex tissues of the pridopidine treated and vehicle treated mice. Next, RNAaseq was performed using the Illumina TruSeq Stranded mRNA Kit with HiSeq 2x50nt paired end sequencing. Fastq files were downloaded, and Star aligner with GRCm38 primary assembly annotation and standard options was used to align the fastq files. Genes were counted with FeatureCounts on GeneCode vM7. For feature_type and group_by, "gene" was used and "reverse" was used for strandedness. Merging of read counts into a single matrix and all other downstream computational processing was done and will be done in R statistical programming language. Plots showing the first and second principal component of the samples were used to select outliers. Transcripts that had less than 10 reads per gene on average were filtered out. CalcNormFactors from the edgeR R package was used to normalize the counts via the TMM method. The limma R-package was used to transform and model the gene-level quantification data. limma::voom was used to transform the count data to log2-counts per million and calculate the meanvariance relationship.

For yet to be completed differential expression analysis, limma::lmFit is used to fit a linear model for each gene based on the experimental design matrix and with an added term to correct for batch information. limma::eBayes is used to calculate the empirical Bayes moderated t-statistic for contrast significance. Multiple hypothesis adjusted p-values ise calculated using limma: :toptable, which implemented the Benjamini-Hochberg procedure to control FDR. Differential expression contrasts between untreated MeCP2 HET and untreated WT samples, treated MeCP2 HET and untreated MeCP2 HET samples, will be independently calculated for all three tissues. To test whether the treatment gene expression signature is enriched for relevant biological signatures, Gene Set Enrichment Analysis (GSEA) is used. Genes are ranked by limma generated t-statistic for a given contrast. Enrichr is used for pathway analysis.

Expected results and potential use

This experiment was designed to assess whether pridopidine reverses aberrant transcription observed in the MeCP2 HET mice. This will be done by taking the list of genes which are perturbed in the disease context, and testing if pridopidine restores expression of these genes to their wild type level. Additionally, answers to the question of what impact pridopidine has on gene expression in the context of a Rett disease mouse model are sought. Pathway analysis may yield relevant pathways, transcription factors, or kinase enrichments that point to pridopidine's potential mechanism of action in RTT. The BDNF pathway, and other relevant pathways will be specifically queried. The signature of pridopidine gene expression in the context of Rett syndrome is used to help develop a pharmacodynamic biomarker (PD biomarker). Therapeutically relevant pridopidine induced gene expression changes observed in the striatum, hippocampus, or cortex may be observable in a more accessible tissue during a clinical trial.

These experiments are performed in order to support the use of pridopidine in treating subjects afflicted with RTT.

## Example 3: Assessment of Efficacy of Pridopidine In Treating Patients Afflicted With RTT

Periodically administering pridopidine (e.g., daily or twice daily) intravenously or orally to a patient afflicted with RTT is effective to treat the patient.

Administering pridopidine effectively delays the onset of symptoms in the RTT patient.

Administering pridopidine effectively prevents or delays the worsening of, or improves at least one symptom in the RTT patient.

Administering pridopidine effectively prevents or delays the worsening of, or improves the mobility skill of the RTT patient. Administering pridopidine effectively prevents a partial or complete loss of acquired mobility skill of the RTT patient.

Administering pridopidine effectively prevents or delays the worsening of, or improves the gait of the RTT patient.

Administering pridopidine effectively prevents, delays or improves ataxia, apraxia, muscle weakness, spasticity, and/or rigidity in the RTT patient. Administering pridopidine effectively prevents, delays or improves impaired gait initiation in the RTT patient.

Administering pridopidine effectively prevents, delays or improves abnormal muscle tone, peripheral vasomotor disturbance, and/or scoliosis in the RTT patient.

Administering pridopidine effectively prevents or delays the worsening of, or improves purposeful hand skills in the RTT patient. Administering pridopidine effectively prevents, delays or improves abnormal hand movement, including but not limited to wringing, squeezing, clapping, washing, tapping, rubbing, and repeatedly bringing hands to mouth. Administering pridopidine effectively prevents a partial or complete loss of acquired purposeful hand skill of the RTT patient.

Administering pridopidine effectively prevents or delays the worsening of, or improves the communication skill of the RTT patient, including but not limited to speech and normal eye contact. Administering pridopidine effectively prevents a partial or complete loss of acquired communication skill of the RTT patient.

Administering pridopidine effectively prevents, delays or improves growth retardation, seizure, cardiac abnormality, breathing irregularity, impaired sleeping pattern, bruxism while awake, decreased response to pain, hypotrophic cold blue feet, increased irritability, decreased alertness, decreased attention span, inappropriate laughing, and/or inappropriate screaming.

## REFERENCES:

Amaral, M.D., et al. (2007) "TRPC channels as novel effectors of BDNF signaling: Potential implications for Rett syndrome". Pharmacol Ther, 113(2):394-409.

CSID:25948790, www.chemspider.com/Chemical-Structure.25948790.html (accessed 23:27, Jul 15, 2016).

CSID:7971505, www.chemspider.com/Chemical-Structure.7971505.html (accessed 23:33, Jul 15, 2016).

Geva , M. et al. Pridopidine activates neuroprotective pathways impaired in Huntington Disease. Human Molecular Genetics, 2016, 25(18):3975-3987

Guy J, Hendrich B, Holmes M, Martin JE, Bird A. (2001) A mouse MeCP2-null mutation causes neurological symptoms that mimic Rett syndrome. Nat Genet. 27(3):322-326.

Isaias, I.U., et al. (2014). "Gait Initiation in Children with Rett Syndrome." PLoS One, 9(4): e92736.

Ponten H, Kullingsjö J, Lagerkvist S, Martin P, Pettersson F, Sonesson C, Waters S, Waters N. In vivo pharmacology of the dopaminergic stabilizer pridopidine. (2010) Eur J Pharmacol. 644(1-3):88-95.

Pozzo-Miller, L., Pati S., & Percy, A.K. (2015). "Rett Syndrome: Reaching for Clinical Trials." Neurotherapeutics, 12(3):631-40.

U.S. Publication No. 2013/0267552 A1 (Teva Pharmaceuticals International GMBH), published October 10, 2013.

U.S. Publication No. 2014/0378508 (Teva Pharmaceuticals International GMBH), published December 25, 2014.

U.S. Publication No. 2015/0202302 (Teva Pharmaceuticals International GMBH), published July 23, 2015.

U.S. Patent No. 7,923,459 (Teva Pharmaceuticals International GMBH), issued April 12, 2011.

U.S. Patent No. 6,903,120 (Teva Pharmaceuticals International GMBH), issued June 7, 2015.

Weng, S.M. et al. (2011). "Rett Syndrome: From Bed to Bench." Pediatrics and Neonatology, 52:309-316.

## Claims

1. A composition comprising pridopidine or its pharmaceutically acceptable salt for use in treating a subject afflicted with Rett syndrome (RTT), wherein the composition treats the subject by delaying the onset, preventing worsening, delaying worsening or improving at least one RTT symptom in the subject, wherein the RTT symptom is abnormal gait, ataxia, impaired gait initiation, a delay in acquiring purposeful hand skills or a partial or complete loss of acquired purposeful hand skills, abnormal hand movement, startle response, delayed crawling and/or walking, or decreased ability to crawl and/or walk.

2. The composition for use according to claim 1, wherein the pridopidine salt is pridopidine hydrochloride, hydrobromide, nitrate, perchlorate, phosphate, sulphate, formate, acetate, aconate, ascorbate, benzenesulphonate, benzoate, cinnamate, citrate, embonate, enantate, fumarate, glutamate, glycolate, lactate, maleate, malonate, mandelate, methanesulphonate, naphthalene-2-sulphonate, phthalate, salicylate, sorbate, stearate, succinate, tartrate or toluene-p-sulphonate salt.

3. The composition for use according to claim 1 or 2, wherein the pridopidine is administered orally, nasally, inhaled, by subcutaneous injection, or through an intravenous, intraperitoneal, intramuscular, intranasal, buccal, vaginal, rectal, intraocular, intrathecal, topical or intradermal route.

4. The composition for use according to any of claims 1-3, wherein the composition is administered in the form of an aerosol, an inhalable powder, an injectable, a liquid, a gel, a solid, a capsule or a tablet.

5. The composition for use according to any of claims 1-4, wherein the pridopidine is administered once daily or twice daily.

6. The composition for use according to any of claims 1-5, wherein the amount of pridopidine administered is 10 mg/day-315 mg/day; or wherein the amount of pridopidine administered in a dose is 10 mg, 22.5 mg, 45 mg, 67.5 mg, 90 mg, 100 mg, 112.5 mg, 125 mg, 135 mg, 150 mg, 180 mg, 200 mg, 250 mg, or 315 mg; or wherein the amount of pridopidine administered in a dose is 10mg-45 mg.

7. The composition for use according to any of claims 1-6, wherein the abnormal hand movement is wringing, squeezing, clapping, washing, tapping, rubbing, and/or repeatedly bringing hands to mouth.

8. The composition for use according to any of claims 1-7, wherein the pridopidine improves the symptom by at least 20%, at least 30%, at least 50%, at least 80%, or 100%.

9. The composition for use according to any of claims 1-8, wherein the pridopidine treats the subject by improving or maintaining the subject's ability to perform activities of daily living, perform domestic chores, manage finances, perform an occupation, and/or wherein the pridopidine treats the subject by reducing the level of nursing care needed by the subject.

10. The composition for use according to any of claims 1-9, wherein the pridopidine increases or maintains the BDNF serum level in the subject and/or increases the BDNF brain levels in the subject.

11. The composition for use according to any of claims 1-10, wherein the subject has a mutation in at least one of the methyl CpG binding protein 2 (MeCP2) gene, the cyclin-dependent kinase-like 5 (CDKL5) gene or the Forkhead box protein G1 (FOXG1) gene.

## Patentansprüche

1. Zusammensetzung, umfassend Pridopidin oder ein pharmazeutisch annehmbares Salz davon zur Anwendung bei der Behandlung eines Subjekts mit Rett-Syndrom (RTT), wobei das Subjekt derart mit der Zusammensetzung behandelt wird, dass das Einsetzen mindestens eines RTT-Symptoms bei dem Subjekt verzögert wird, dass gegen ein Verschlechtern eines solchen vorgebeugt wird, dass ein Verschlechtern eines solchen verzögert wird oder dass ein solches verbessert wird, wobei das RTT-Symptom ein pathologischer Gang, Ataxie, eine gestörte Gangeinleitung, ein verspäteter Erwerb zweckgerichteter Handgeschicklichkeit oder ein teilweiser oder vollständiger Verlust erworbener zweckgerichteter Handgeschicklichkeit ist sowie pathologische Handbewegungen, Schreckreaktion, verspätetes Kriechen und/oder Laufen oder verminderte Kriech- und/oder Lauffähigkeit ist/sind.

2. Zusammensetzung zur Anwendung nach Anspruch 1, wobei das Salz von Pridopidin Pridopidinhydrochlorid-, -hydrobromid-, -nitrat-, -perchlorat-, -phosphat-, -sulfat-, -format-, - acetat-, -akonat-, -ascorbat-, -benzolsulfonat-, -benzoat-, -cinnamat-, -citrat-, -embonat-, -enantat, -fumarat-, -glutamat-, -glycolat-, -lactat-, -maleat-, -malonat-, -mandelat-, -methansulfonat-, - naphthalen-2-sulfonat-, -phthalat-, -salicylat-, -sorbat-, -stearat-, -succinat-, -tartrat- oder -toluolp-sulfonatsalz ist.

3. Zusammensetzung zur Anwendung nach Anspruch 1 oder 2, wobei das Pridopidin oral, nasal, durch Inhalation, durch subkutane Injektion oder über einen intravenösen, intraperitonealen, intramuskulären, intranasalen, bukkalen, vaginalen, rektalen, intraokulären, intrathekalen, topischen oder intradermalen Weg verabreicht wird.

4. Zusammensetzung zur Anwendung nach einem der Ansprüche 1-3, wobei die Zusammensetzung in der Form eines Aerosols, eines inhalierbaren Pulvers, einer injizierbaren Substanz, einer Flüssigkeit, eines Gels, eines Feststoffs, einer Kapsel oder einer Tablette verabreicht wird.

5. Zusammensetzung zur Anwendung nach einem der Ansprüche 1-4, wobei das Pridopidin einmal oder zweimal täglich verabreicht wird.

6. Zusammensetzung zur Anwendung nach einem der Ansprüche 1-5, wobei die Menge von verabreichtem Pridopidin 10 mg/Tag-315 mg/Tag beträgt; oder wobei die Menge von verabreichtem Pridopidin in einer Dosis von 10 mg, 22,5 mg, 45 mg, 67,5 mg, 90 mg, 100 mg, 112,5 mg, 125 mg, 135 mg, 150 mg, 180 mg, 200 mg, 250 mg oder 315 mg vorliegt; oder wobei die Menge von verabreichtem Pridopidin in einer Dosis von 10 mg bis 45 mg vorliegt.

7. Zusammensetzung zur Anwendung nach einem der Ansprüche 1-6, wobei die pathologische Handbewegung ein Wringen, Zusammendrücken, Zusammenklatschen, Waschen, Aufschlagen, Reiben und/oder wiederholtes Bringen der Hände zum Mund ist.

8. Zusammensetzung zur Anwendung nach einem der Ansprüche 1-7, wobei das Pridopidin das Symptom zu mindestens 20 %, mindestens 30 %, mindestens 50 %, mindestens 80 % oder 100 % verbessert.

9. Zusammensetzung zur Anwendung nach einem der Ansprüche 1-8, wobei das Pridopidin das Subjekt durch ein Verbessern oder Aufrechterhalten der Fähigkeit des Subjekts, Aktivitäten der täglichen Lebensführung wahrzunehmen, Aufgaben der Haushaltsführung zu verrichten, Finanzen zu verwalten, einen Beruf auszuüben, behandelt; und/oder wobei das Pridopidin das Subjekt durch eine Reduzierung des benötigten Pflegeaufwands behandelt.

10. Zusammensetzung zur Anwendung nach einem der Ansprüche 1-9, wobei das Pridopidin den BDNF-Spiegel in Serum des Subjekts erhöht oder aufrechterhält und/oder den BDNF-Spiegel im Gehirn des Subjekts erhöht.

11. Zusammensetzung zur Anwendung nach einem der Ansprüche 1-10, wobei das Subjekt eine Mutation in mindestens einem der folgenden Gene hat: Methyl-CpG-Protein 2 (MeCP2), Cyclinabhängige kinase-ähnliche 5 (CDKL5) oder Forkhead-Box-Protein G1 (FOXG1).

**Revendications**

1. Composition comprenant de la pridopidine ou son sel pharmaceutiquement acceptable destinée à une utilisation dans le traitement d'un sujet atteint du syndrome de Rett (RTT), dans laquelle la composition traite le sujet en retardant l'apparition, en empêchant l'aggravation, en retardant l'aggravation ou en améliorant au moins un symptôme du RTT chez le sujet, le symptôme du RTT étant une démarche anormale, une ataxie, une altération de l'initiation de la démarche, un retard dans l'acquisition d'habiletés manuelles ciblées ou une perte partielle ou complète des habiletés manuelles ciblées acquises, un mouvement anormal de la main, une réaction de sursaut, un retard du rampement et/ou de la marche, ou une diminution de la capacité de ramper et/ou de marcher.

2. Composition destinée à une utilisation selon la revendication 1, dans laquelle le sel de pridopidine est le chlorhydrate, le bromhydrate, le nitrate, le perchlorate, le phosphate, le sulfate, le formiate, l'acétate, l'aconate, l'ascorbate, le benzènesulfonate, le benzoate, le cinnamate, le citrate, l'embonate, l'énantate, le fumarate, le glutamate, le glycolate, le lactate, le maléate, le malonate, le mandélate, le méthanesulfonate, le naphtalène-2-sulfonate, le phtalate, la salicylate, le sorbate, le stéarate, le succinate, le tartrate ou le sel de toluène-p-sulfonate.

3. Composition destinée à une utilisation selon la revendication 1 ou 2, dans laquelle la pridopidine est administrée par voie orale, nasale, inhalée, par injection sous-cutanée, ou par voie intraveineuse, intrapéritonéale, intramusculaire, intranasale, buccale, vaginale, rectale, intraoculaire, intrathécale, topique ou intradermique.

4. Composition destinée à une utilisation selon l'une quelconque des revendications 1 à 3, dans laquelle la composition est administrée sous la forme d'un aérosol, d'une poudre inhalable, d'un produit injectable, d'un liquide, d'un gel, d'un solide, d'une capsule ou d'un comprimé.

5. Composition destinée à une utilisation selon l'une quelconque des revendications 1 à 4, dans laquelle la pridopidine est administrée une fois par jour ou deux fois par jour.

6. Composition destinée à une utilisation selon l'une quelconque des revendications 1 à 5, dans laquelle la quantité de pridopidine est administrée en une dose de 10 mg/jour à 315 mg/jour ; ou dans laquelle la quantité de pridopidine est administrée en une dose de 10 mg, 22,5 mg, 45 mg, 67,5 mg, 90 mg, 100 mg, 112,5 mg, 125 mg, 135 mg, 150 mg, 180 mg, 200 mg, 250 mg, ou de 315 mg ; ou dans laquelle la quantité de pridopidine est administrée en une dose de 10 mg à 45 mg.

7. Composition destinée à une utilisation selon l'une quelconque des revendications 1 à 6, dans laquelle le mouvement anormal de la main consiste à tordre, serrer, taper des mains, laver, tapoter, frotter et/ou porter les mains à la bouche de manière répétée.

8. Composition destinée à une utilisation selon l'une quelconque des revendications 1 à 7, dans laquelle la pridopidine améliore le symptôme d'au moins 20 %, d'au moins 30 %, d'au moins 50 %, d'au moins 80 % ou de 100 %.

9. Composition destinée à une utilisation selon l'une quelconque des revendications 1 à 8, dans laquelle la pridopidine traite le sujet en améliorant ou en maintenant la capacité du sujet à effectuer des activités de la vie quotidienne, à effectuer des tâches ménagères, à gérer ses finances, à exercer une profession, et/ou dans laquelle la pridopidine traite le sujet en réduisant le niveau de soins infirmiers requis par le sujet.

10. Composition destinée à une utilisation selon l'une quelconque des revendications 1 à 9, dans laquelle la pridopidine augmente ou maintient le taux sérique de BDNF chez le sujet et/ou augmente les taux cérébraux de BDNF chez le sujet.

11. Composition destinée à une utilisation selon l'une quelconque des revendications 1 à 10, dans laquelle le sujet a une mutation dans au moins l'un du gène de la protéine de liaison au méthyle CpG 2 (MeCP2), du gène cycline-dépendante de la kinase 5 (CDKL5) ou du gène de la protéine Forkhead box G1 (FOXG1).

**Figure 1**

**Figure 2**

**Figure 3**

**Figure 4**

**Figure 5**

**Figure 6**

**Figure 7**

**Figure 8A**

**Figure 8B**

percent recovery

**Figure 9A**

**Figure 9B**

percent recovery

**Figure 10A**

**Figure 10B**

**Figure 10C**

**Figure 11A**

**Figure 11B**

**Figure 12A**

**Figure 12B**

**Figure 13A**

**Figure 13B**

**Figure 14A**

**Figure 14B**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015112601 A1
- WO 2013086425 A1
- WO 0146145 A1
- US 20130197031
- US 20160166559 A
- US 20160095847 A
- US 20130267552 A1
- US 20140378508 A
- US 20150202302 A
- US 7923459 B
- US 6903120 B
- WO 62395854 A

### Non-patent literature cited in the description

- *CHEMICAL ABSTRACTS,* 346688-38-8
- **AMARAL, M.D. et al.** TRPC channels as novel effectors of BDNF signaling: Potential implications for Rett syndrome. *Pharmacol Ther,* 2007, vol. 113 (2), 394-409
- **GEVA , M. et al.** Pridopidine activates neuroprotective pathways impaired in Huntington Disease. *Human Molecular Genetics,* 2016, vol. 25 (18), 3975-3987
- **GUY J ; HENDRICH B ; HOLMES M ; MARTIN JE ; BIRD A.** A mouse MeCP2-null mutation causes neurological symptoms that mimic Rett syndrome. *Nat Genet,* 2001, vol. 27 (3), 322-326
- **ISAIAS, I.U. et al.** Gait Initiation in Children with Rett Syndrome. *PLoS One,* 2014, vol. 9 (4), e92736
- **PONTEN H ; KULLINGSJÖ J ; LAGERKVIST S ; MARTIN P ; PETTERSSON F ; SONESSON C ; WATERS S ; WATERS N.** In vivo pharmacology of the dopaminergic stabilizer pridopidine. *Eur J Pharmacol,* 2010, vol. 644 (1-3), 88-95
- **POZZO-MILLER, L. ; PATI S. ; PERCY, A.K.** Rett Syndrome: Reaching for Clinical Trials. *Neurotherapeutics,* 2015, vol. 12 (3), 631-40
- **WENG, S.M. et al.** Rett Syndrome: From Bed to Bench. *Pediatrics and Neonatology,* 2011, vol. 52, 309-316